# EUROPÄISCHE PATENTSCHRIFT

(11) **EP 3 793 665 B1**
(45) Veröffentlichungstag und Bekanntmachung des Hinweises auf die Patenterteilung: **03.07.2024**
(21) Anmeldenummer: 19723062.6
(22) Anmeldetag: 07.05.2019
(51) Int. Cl.: A61N 1/05

(54) **VORRICHTUNG ZUR EXTRAVASALEN ODER EXTRANEURONALEN BEFESTIGUNG EINES MEDIZINISCHEN IMPLANTATS IN ART EINER WICKELMANSCHETTE**
DEVICE FOR EXTRAVASCULAR OR EXTRANEURONAL FASTENING OF A MEDICAL IMPLANT IN THE MANNER OF A COMPRESSION SLEEVE
DISPOSITIF DE FIXATION EXTRAVASAL OU EXTRANEURONAL D'UN IMPLANT MÉDICAL A LA MANIÈRE D'UN BRASSARD

(30) Priorität: 17.05.2018 DE 102018207709
(43) Veröffentlichungstag der Anmeldung: 24.03.2021
(73) Patentinhaber: Neuroloop GmbH, 79110 Freiburg (DE)
(72) Erfinder: PLACHTA, Dennis, 79279 Vörstetten (DE); BORETIUS, Tim, 79098 Freiburg (DE); KIMMIG, Fabian, 79110 Freiburg (DE); HASSLER, Christina, 79276 Reute (DE)
(74) Vertreter: Rösler, Uwe
(86) Internationale Anmeldenummer: PCT/EP2019/061599
(87) Internationale Veröffentlichungsnummer: WO 2019/219436

(56) Entgegenhaltungen:
- WO-A1-2016/055512
- DE-U1-202007 019 439
- US-A- 4 341 221
- US-A1- 2003 040 785
- US-A1- 2014 188 202

## Beschreibung

### Technisches Gebiet

Die Erfindung bezieht sich auf eine Vorrichtung zur extravasalen oder extraneuronalen Befestigung eines medizinischen Implantats mit einem biokompatiblen Flächensubstrat, das einen in Art einer Wickelmanschette ausgebildeten ersten Substratabschnitt aufweist, der über ein freies Abschnittsende verfügt, das durch Wickeln des ersten Substratabschnittes um eine Raumachse wenigstens einlagig lose von dem gewickelten ersten Substratabschnitt radial überdeckt ist, sowie einen sich einstückig an den ersten Substratabschnitt anschließenden zweiten, nicht um die Raumachse gewickelten Substratabschnitt vorsieht, der mittel- oder unmittelbar mit einer von dem medizinischen Implantat wegführenden Verbindungstruktur verbindbar ist.

### Stand der Technik

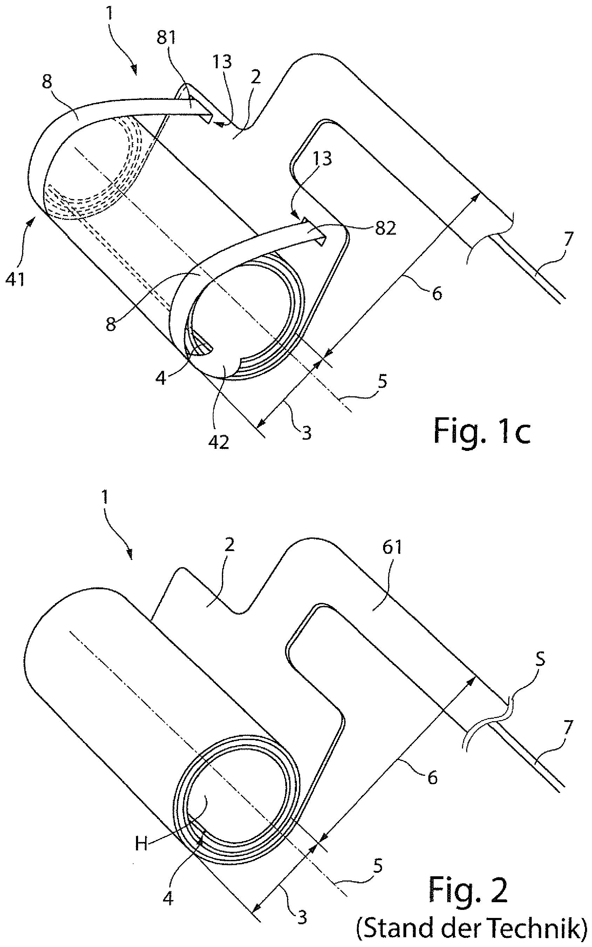

Ein gattungsgemäßes medizinisches Implantat, das in Art einer Wickelmanschette zum Zwecke der Detektion sowie auch Applikation neuronaler elektrischer Signale für einen dauerhaften oder zumindest langfristigen Verbleib längs eines Nervenfaserbündels innerhalb des menschlichen oder tierischen Körpers geeignet ausgebildet ist, ist in der Druckschrift WO 2016/055512 A1 beschrieben sowie in Figur 2 schematisiert dargestellt. Das aus einem biokompatiblen Flächensubstrat 2 gefertigte und als Wickelmanschette ausgebildete medizinische Implantat 1 weist einen ersten Substratabschnitt 3 auf, der um eine Raumachse 5 unter Ausbildung wenigstens einer Flächensubstratwicklung gewickelt ist, die einen geradzylinderförmigen Hohlraum H radial umfasst. An der dem geradzylindrigen Hohlraum H zugewandten Flächensubstratoberfläche ist eine Vielzahl nicht in Figur 2 dargestellter Elektrodenflächen angebracht, die in körperlichem Kontakt mit dem Epineurium eines von der Wickelmanschette 1 umfassten Nervenfaserbündels tritt, ebenfalls in Figur 2 nicht dargestellt. Um zu gewährleisten, dass das medizinische Implantat 1 einerseits möglichst ortsfest längs eines Nervenfaserbündels nach entsprechender Implantation verbleibt und andererseits den natürlichen Formänderungen eines Nervenfaserbündels zu folgen vermag oder diesem zumindest keinen nennenswerten mechanischen Widerstand entgegensetzt, liegen die einzelnen Flächensubstratwicklungen lose aneinander an und vermögen im Falle einer Ausdehnung des Nervenfaserbündels durch entsprechende Relativbewegung den Durchmesser des umfassten Hohlraumes H zu vergrößern.

Durch eine dem Flächensubstrat 2 im ersten Substratabschnitt 3 eingeprägte Materialvorspannung nimmt das Flächensubstrat 2 ohne äußere Krafteinwirkung eine vordefinierte Wickelkonfiguration ein, bei der das freie Abschnittsende 4 wenigstens einlagig lose von dem ersten Substratabschnitt, d.h. von wenigstens einer Flächensubstratwicklung radial überdeckt ist.

An dem gewickelten ersten Substratabschnitt 3 schließt einstückig ein nicht um die Raumachse 5 gewickelter zweiter Substratabschnitt 6 an, innerhalb dem elektrische Zuleitungen geführt sind, die zu der Vielzahl der mit dem Epineurium des Nervenfaserbündels in Kontakt tretenden Elektroden verbunden sind. Der gleichfalls flächig ausgebildete zweite Substratabschnitt 6 weist im dargestellten Ausführungsbeispiel einen parallel zur Raumachse 5 orientierten stegförmig ausgebildeten Flächenabschnitt 6 auf, der über eine nicht weiter dargestellte Schnittstelle S, beispielsweise in Form einer implantierbaren Steckerverbindung mit einer von dem medizinischen Implantat 1 wegführenden Verbindungsstruktur 7 verbunden ist, längs der die elektrischen Leitungen zu einer separaten, vorzugsweise implantierbaren Versorgungseinheit geführt werden.

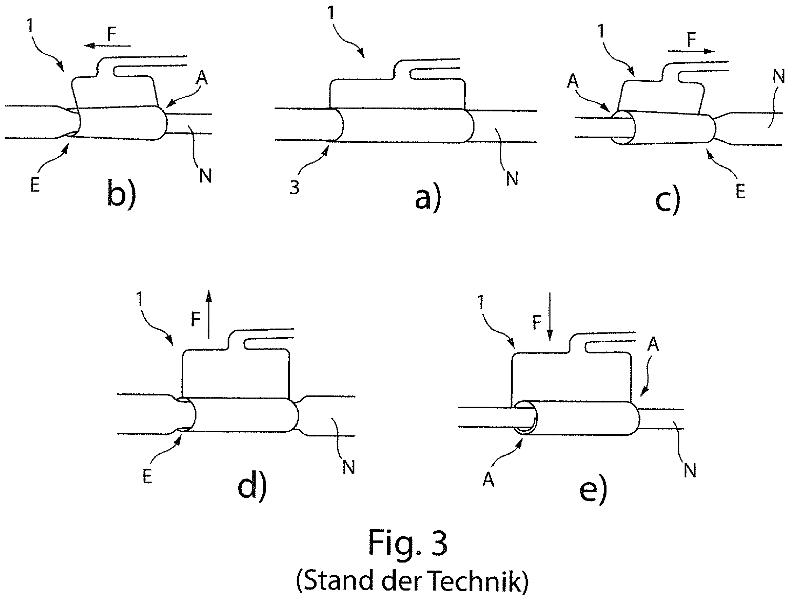

In einem unbelasteten Zustand sitzt das medizinische Implantat 1 längs eines Nervenfaserbündels N gemäß der in Figur 3a schematisierten Darstellung mit einer im Durchmesser einheitlichen Umfassung des zu einer Wickelmanschette geformten ersten Substratabschnittes 3 an. In diesem Zustand wirkt kein oder nur ein minimaler mechanischer äußerer Zwang auf das Nervenfaserbündel N ein. Wirken hingegen externe Kräfte F auf das medizinische Implantat 1 ein, die beispielsweise von körpereigenen Bewegungen herrühren, so kann es zu Verformungen der Wickelgeometrie längs des als Wickelmanschette ausgebildeten ersten Substratabschnittes 3 kommen, wodurch zum einen die Befestigung des medizinischen Implantates längs des Nervenfaserbündels N nicht mehr sichergestellt sein kann und zum anderen ein mechanischer Stress durch das medizinische Implantat auf das Nervenfaserbündel einwirken kann. In den Figuren 3b bis e sind derartige Belastungssituationen skizziert. So führen beispielsweise Zugkräfte F, die im Wesentlichen parallel zur Längserstreckung des Nervenfaserbündels N orientiert sind, innerhalb der Wickelmanschette zu einer trichterförmigen Verformung, die einerseits zu einer Einschnürung E am Nervenfaserbündel N und andererseits zu einer Aufweitung A und damit verbundenen radialen Beabstandung der Wickelmanschette vom Nervenfaserbündel N führen, siehe hierzu Figuren 3b und c. Zu einer Einschnürung E des Nervenfaserbündels N kann es ebenfalls im Falle einer orthogonal zur Nervenfaserbündellängserstreckung orientierten Krafteinwirkung F auf das medizinische Implantat 1 kommen, siehe die in Figur 3d illustrierte Belastungssituation. In diesem Fall wirkt die Kraft F als Zugkraft quer zum Nervenfaserbündel N. In Figur 1e wirkt die Kraft F in umgekehrter Kraftrichtung, in Form einer auf das Nervenfaserbündel N gerichteten Schubkraft, wodurch die Wickelmanschette im Durchmesser aufgeweitet und vom Nervenfaserstrang N tendenziell gelöst wird.

Eine Manschettenelektrode in einer ursprünglichen Form ist in der DE 44 33 111 A1 dargestellt, die über eine interdigitale Flächenform verfügt, deren einzelne Fingerabschnitte je nach äußeren Druck- und Kraftbelastungen in sich öffnender Weise nachgibt.

Die Druckschrift US 4602624 offenbart eine implantierbare Cuff-Elektrode mit einem zu einer Hohlzylinderform selbstaufwickelnden Flächensubstrat, das über unterschiedliche Innendurchmesser in axialer Längserstreckung verfügt. Die Hohlzylinderform wird ausschließlich durch Material-inhärent vorgegebene Vorspannungen aufrechterhalten.

Die Druckschrift WO 2013/150524 A1 offenbart eine Elektroden Cuff-Anordnung mit einer Reihe von als Elektroden dienenden einzelnen Metallmanschetten, die gesamtheitlich radial von außen von einem Mantelgehäuse umfasst sind, das aus einem geöffneten in einen geschlossenen Zustand überführbar ist, letzterer wird mittels eines Verschlussklammermechanismus gesichert.

Die Druckschrift US2003/0040785 A1 offenbart eine implantierbare Elektrodenanordnung, die einseitig auf einer flexiblen Trägerfolie aufgebracht und zu einem Hohlzylinder formbar ist, wobei zwei sich gegenüberliegende Seitenkanten der Trägerfolie aneinandergrenzen und die implantierbare Elektrodenanordnung mittels eines geeignet ausgebildeten Schließmechanismus, der an den zwei Seitenkanten angebracht ist, im Zustand der Hohlzylinderform gehalten wird.

Die Druckschrift US2014/0188202 A1 beschreibt eine Wickelelektrodenanordnung mit einem durch materialinhärente Vorspannung einen Nervenstrang selbstständig und radial vollständig umfassenden Flächensubstrat, an dessen zwei Flächensubstratenden jeweils eine Handhabungseinheit angebracht ist, mit der das Flächensubstrat aus dem selbstständig eingenommenen gewickelten Zustand in einen gestreckten Zustand überführbar ist.

### Darstellung der Erfindung

Die Erfindung wird vom Anspruch 1 definiert.

Der Erfindung liegt die Aufgabe zugrunde eine Vorrichtung zur extravasalen oder extraneuronalen Befestigung eines medizinischen Implantats mit einem biokompatiblen Flächensubstrat, das einen in Art einer Wickelmanschette ausgebildeten ersten Substratabschnitt, der ein freies Abschnittsende verfügt, das durch Wickeln des ersten Substratabschnittes um eine Raumachse wenigstens einlagig lose von dem gewickelten ersten Substratabschnitt radial überdeckt ist, sowie einen sich einstückig an den ersten Substratabschnitt anschließenden zweiten, nicht um die Raumachse gewickelten Substratabschnitt aufweist, der mittel- oder unmittelbar mit einer von dem medizinischen Implantat wegführenden Verbindungsstruktur verbindbar ist, derart weiterzubilden, so dass eine sichere und zugleich schonende und dauerhafte Anbringung des medizinischen Implantates längs eines intrakorporalen Gefäßes oder Nervenfaserbündels möglich wird. Die zu treffenden Maßnahmen sollen bewegungsbedingte Verformungen der Wickelmanschette vermeiden oder zumindest in einem Maße reduzieren, so dass keine nennenswerten mechanischen Belastungen auf intrakorporalen Gefäßes oder Nervenfaserbündels ausgeübt werden. Die zu treffenden Maßnahmen sollen für den Operateur im Rahmen der Implantation keinen nennenswerten zeitlichen sowie auch handhabungstechnischen Zusatzaufwand darstellen.

Die Lösung der der Erfindung zugrundeliegenden Aufgabe ist im Anspruch 1 angegeben. Den Lösungsgedanken in vorteilhafter Weise weiterbildende Merkmale sind Gegenstand der Unteransprüche sowie der weiteren Beschreibung insbesondere unter Bezugnahme auf konkrete Ausführungsbeispiele zu entnehmen.

Lösungsgemäß zeichnet sich eine Vorrichtung zur extravasalen oder extraneuronalen Befestigung eines medizinischen Implantates mit einem biokompatiblen Flächensubstrat, das in Art einer Wickelmanschette ausgebildet ist und die Merkmale des Oberbegriffes des Anspruches 1 aufweist, dadurch aus, dass im Bereich des Abschnittsendes des zu einer Wickelmanschette ausgebildeten ersten Substratabschnittes wenigstens ein Mittel angebracht ist, das im gewickelten Zustand des ersten Substratabschnittes um die Raumachse mit dem ersten Substratabschnitt und/oder den zweiten Substratabschnitt räumlich getrennt vom Abschnittsende eine Fügeverbindung eingeht.

Das freie Abschnittsende des zu einer Wickelmanschette gewickelten ersten Substratabschnittes ist lösungsgemäß über ein zumindest Zugkräfte übertragendes Mittel mit einem Bereich des Trägersubstrates lösbar fest verbunden, der sich vorzugsweise innerhalb des zweiten Substratbereiches befindet, also jenem Bereich des Trägersubstrates, der sich einstückig an den in Art einer Wickelmanschette ausgebildeten ersten Substratabschnitt anschließt. Das wenigstens eine Mittel ist derart angeordnet und ausgebildet, so dass das Mittel bei einer äußeren auf das medizinische Implantat einwirkenden Kraft eine Halte- bzw. Stützkraft erzeugt, durch die die Form und Gestalt der Wickelmanschette unverändert erhalten bleibt. Das wenigstens eine Mittel vermag vornehmlich Zugkräfte zwischen dem freien Abschnittsende und dem Fügebereich zu übertragen. Grundsätzlich ist es ebenso denkbar, den Fügebereich innerhalb des ersten Substratabschnittes vorzusehen, jedoch ist in diesem Fall darauf zu achten, dass sich zwischen dem Bereich des Abschnittsendes, an dem das einseitig Mittel angebracht ist, und der Fügeverbindung wenigstens eine vollständige Wicklung des ersten Substratabschnittes um die Raumachse erstreckt.

In einer bevorzugten Ausführungsform ist das wenigstens eine Mittel in Art eines flexiblen und nicht dehnbaren Stranggutes, vorzugsweise in Art eines Fadens oder Bandes ausgebildet. Das Stranggut ist jeweils mit einem seiner beiden Stranggutenden fest im Bereich des Abschnittsendes des ersten Substratabschnittes gefügt oder mit diesem einstückig verbunden. Das andere Ende des Stranggutes ist im Bereich des ersten und/oder zweiten Substratabschnittes im Wege einer lösbar festen Fügeverbindung, vorzugsweise in Form einer Kraft- und/oder Formschlussverbindung befestigt. In vorteilhafter Weise weist hierzu das biokompatible Flächensubstrat im Bereich des ersten und/oder zweiten Substratabschnittes wenigstens eine als Flächensubstrat vollständig durchsetzende Befestigungsöffnung auf, durch die das Stranggut zum Zwecke einer lösbar festen Verbindung, vorzugsweise zur Knotenbildung, hindurchfädelbar ist.

In einer weiteren Ausführungsform ist das Mittel in Art einer Lasche ausgebildet, die den ersten Substratabschnitt im Bereich des Abschnittsendes seitlich überragt und einstückig mit diesem verbunden ist. Längs des ersten oder zweiten Substratabschnittes ist eine schlitzförmige Ausnehmung vorgesehen, in die die Lasche unter Ausbildung einer Kraft- und/oder formschlüssigen Fügeverbindung fügbar ist. Hierbei befindet sich vorzugsweise die schlitzförmige Ausnehmung innerhalb eines Bereiches des ersten oder zweiten Substratabschnittes, der den zu einer Wickelmanschette ausgebildeten ersten Substratabschnitt in Wickellängserstreckung überragt. Eine nähere Erläuterung hierzu ist der nachfolgenden Beschreibung unter Bezugnahme auf ein konkretes Ausführungsbeispiel zu entnehmen.

Grundsätzlich weist der erste Substratabschnitt eine sich um die Raumachse orientierte Wickellängserstreckung sowie eine axial zur Raumachse orientierte Manschettenlängserstreckung auf, wobei die Wickellängserstreckung durch den Abstand zwischen dem freien Abschnittsende und dem an den ersten Substratabschnitt angrenzenden zweiten Substratabschnitt bestimmt ist. In einer bevorzugten Ausführungsform verjüngt sich die Manschettenlängserstreckung innerhalb des ersten Substratabschnittes mit zunehmendem Abstand zum Bereich des Abschnittsendes längs der Wickelerstreckung stufenweise, vorzugsweise gleichmäßig. Hierdurch bleiben die sich gegenüberliegenden Enden des Abschnittsendes in der gewickelten Form des ersten Substratabschnittes axial von außen frei zugänglich, so dass sich das Stranggut ungehindert von den sich jeweils gegenüberliegenden Enden des Abschnittendes bis zum Fügebereich erstrecken kann.

Weitere lösungsgemäße Aspekte und Merkmale sind der weiteren Beschreibung unter Bezugnahme auf die Figuren zu entnehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a: lösungsgemäßes Ausführungsbeispiel mit einem einzigen Verbindungsmittel,
- Fig. 1b,c: lösungsgemäße Ausführungsbeispiele mit jeweils zwei Verbindungsmitteln,
- Fig. 2: medizinisches Implantat mit Wickelmanschette gemäß Stand der Technik,
- Fig. 3a-e: diverse Belastungszustände eines medizinischen Implantates in Art einer Wickelmanschette,
- Fig. 4a,c: lösungsgemäßes Ausführungsbeispiel mit beidseitiger Laschenbefestigung sowie
- Fig. 5a,b: lösungsgemäße Ausführungsform mit beidseitig frei zugänglichen Endbereichen längs des freien Abschnittsendes.

### Wege zur Ausführung der Erfindung, gewerbliche Verwendbarkeit

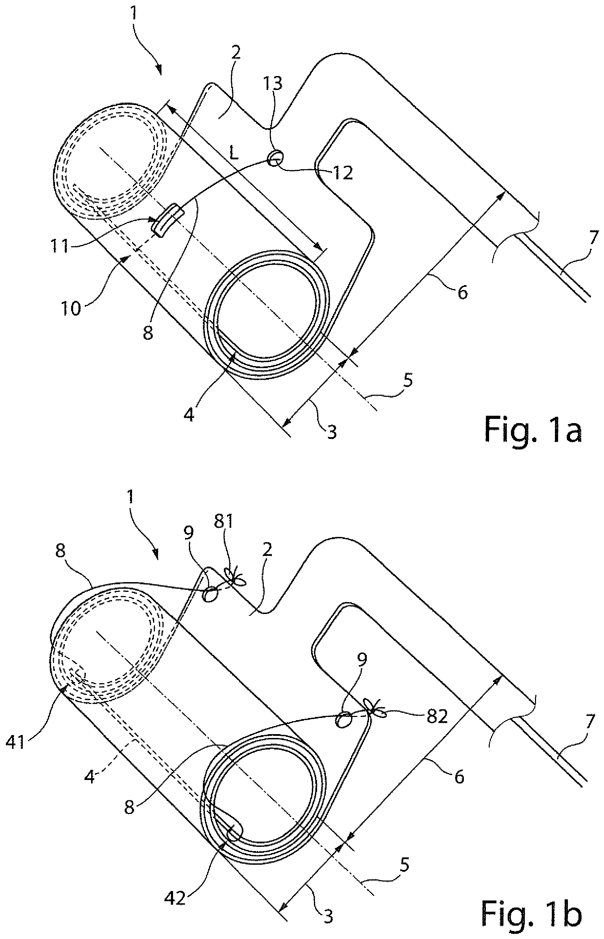

Figur 1a zeigt ein medizinisches Implantat 1 mit einem Flächensubstrat 2, dessen erster Substratabschnitt 3 eine Wickelmanschette bildet, d.h. das freie Abschnittsende 4 des ersten Substratabschnittes 3 ist um die Raumachse 5 unter Ausbildung wenigstens einer Wicklung gewickelt. In Figur 1a sind 2 ½ Wicklungen realisiert. Ein zweiter, nicht gewickelter Substratabschnitt 6 schließt sich einstückig an den ersten Substratabschnitt 3 an.

Mittig längs des freien Abschnittsendes 4 ist ein Ende eines Stranggutes 8, vorzugsweise in Form eines Fadens, Bandes oder eines vergleichbaren Zugkräfte übertragenden Mittels, fest angebracht. Die feste Verbindung 10 ist in Form einer stoff-, kraft- und/oder formschlüssigen, nicht lösbaren Verbindung ausgebildet. Ausgehend vom Ort der Verbindung 10 verläuft das Stranggut 8 durch innerhalb des ersten Substratabschnittes 3 eingebrachte Öffnungen 11, die sich im dargestellten gewickelten Zustand radial deckungsgleich überlappen. Auf diese Weise traversiert das Stranggut 8 die Wickelmanschette von innen nach außen. Das der Verbindung 10 gegenüber liegende Stranggutende ist im Rahmen einer lösbar festen Fügeverbindung 12 mit dem zweiten Substratabschnitt 6 lokal verbunden. Im Bereich der Fügeverbindung 12 ist am Flächensubstrat 2 eine Öffnung 13 vorgesehen, durch die das Stranggut 8 hindurchgeführt und im Wege, vorzugsweise einer Verknotung mit dem zweiten Substratabschnitt 6 verbunden wird.

Durch die, längs der Manschettenlängserstreckung L mittige Anbringung des Stranggutes 8 am Abschnittsende 4 werden Zugkräfte längs des Stranggutes 8 symmetrisch auf das Abschnittsende 4 übertragen und ein Entrollen der Wickelmanschette verhindert. Durch die symmetrische Kraftübertragung werden überdies asymmetrische Belastungszustände, wie sie in den Figuren 3b und c illustriert sind, vermieden.

Das in Figur 1b illustrierte Ausführungsbeispiel sieht anstelle eines einzigen Stranggutes zwei separate Stranggüter 8, jeweils in Form eines chirurgischen Fadens vor, die einseitig an den Enden 41, 42 des freien Abschnittsendes 4 des ersten Substratabschnittes 3 fest gefügt sind. Beide Stranggüter 8 sind an ihren freien Enden 81, 82 über lösbar feste Fügeverbindungen 9 am zweiten Substratabschnitt 6 angebracht. Auch in diesem Fall sind zur Ausbildung der Fügeverbindungen 9 jeweils das Flächensubstrat 2 durchsetzende Öffnungen im zweiten Substratabschnitt 6 vorgesehen.

Figur 1c illustriert ein Ausführungsbeispiel, das anstelle zweier fadenförmiger Stranggüter jeweils zwei bandförmige Stranggüter 8 vorsieht, die jeweils einstückig an den Enden 41, 42 des freien Abschnittsendes 4 des ersten Substratabschnittes 3 verbunden sind. Die gegenüberliegenden Bandenden 81, 82 münden in entsprechende schlitzförmige Ausnehmungen 13 innerhalb des Flächensubstrates 2 des zweiten Substratabschnittes 6 und sind mit diesem lösbar fest verbunden.

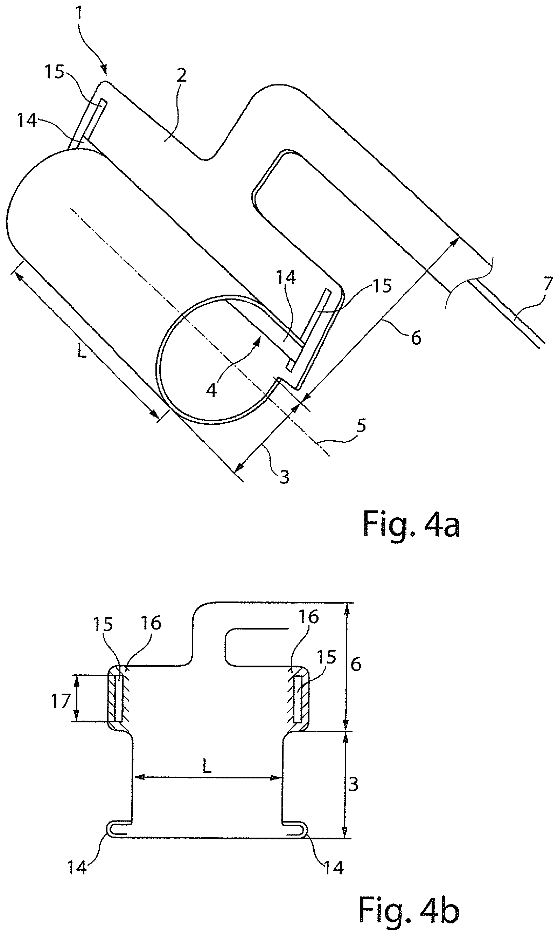

Figuren 4a und b zeigen ein medizinisches Implantat 1 in perspektivischer Schrägansicht, siehe a), sowie in einem nicht aufgewickelten Zustand in Draufsicht, siehe b). In diesem Fall weist das Abschnittsende 4 des ersten Substratabschnittes 3 jeweils zwei den ersten Substratabschnitt 3 seitlich überragende Laschen 14 auf, die im gewickelten Zustand des ersten Substratabschnittes 3 in jeweils am zweiten Substratabschnitt 6 angebrachte schlitzförmige Ausnehmungen 15 unter Ausbildung einer lösbar festen Kraft- und Formschlussverbindung einmünden. Die schlitzförmigen Ausnehmungen 15 befinden sich in jeweils seitlichen Bereichen 16 des zweiten Substratabschnittes 6, siehe schraffierte Flächen, die die Manschettenlängserstreckung L beidseitig überragen. Die mit den schlitzförmigen Ausnehmungen 5 in Eingriff stehenden Laschen 14 vermeiden, gleichsam wie die faden- oder bandförmigen Stranggüter 8, ein unkontrolliertes Loslösen der Wickelmanschette von einem Nervenfaserbündel. Zugleich weisen die schlitzförmigen Ausnehmungen 15 eine Schlitzlänge 17 auf, die im Vergleich zur Laschenbreite etwas größer dimensioniert ist, so dass die Laschen 14 längs der schlitzförmigen Ausnehmungen 15 begrenzt beweglich sind und die Wickelmanschette den natürlichen Verformungen bzw. Ausdehnungen eines Nervenfaserbündels folgen kann.

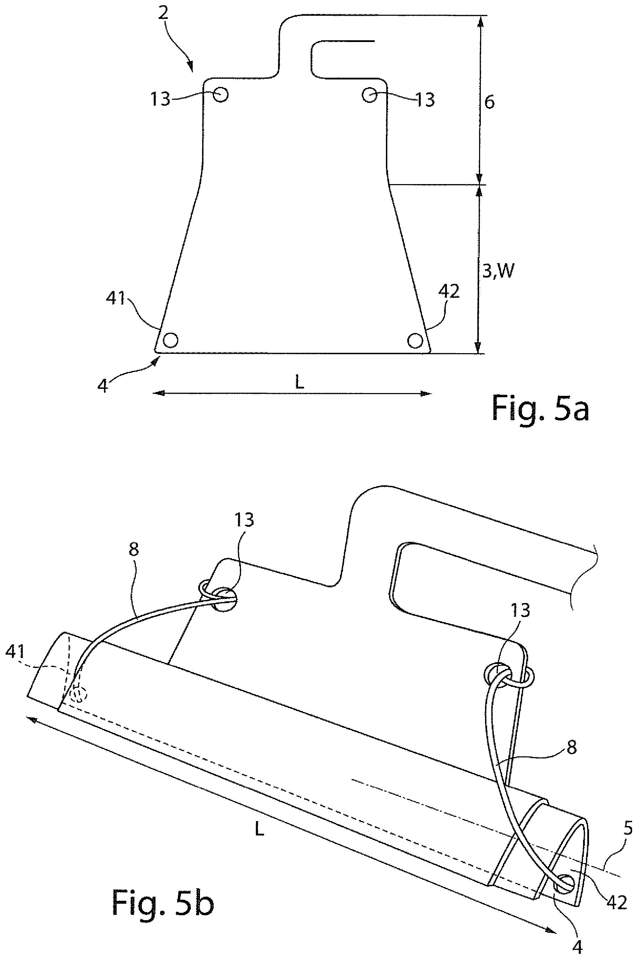

Die Figuren 5a, b stellen ein alternatives Ausführungsbeispiel zur Ausbildung der lösungsgemäßen Befestigungsvorkehrung dar. Figur 5a zeigt eine Draufsicht auf das Flächensubstrat 2 der Wickelmanschette im nicht gewickelten Zustand, Figur 5b zeigt die perspektivische Ansicht auf das medizinische Implantat in Form einer Wickelmanschette. Der erste Substratabschnitt 3 ist trapezförmig derart ausgebildet ist, so dass die Manschettenlängserstreckung L innerhalb des ersten Substratabschnittes 3 mit zunehmendem Abstand zum Bereich des Abschnittsendes 4 längs der Wickelerstreckung W gleichmäßig verjüngt, siehe hierzu Draufsicht auf das nicht gewickelte Flächensubstrat 3 gemäß Figur 5a. Durch Wickeln des ersten Substratabschnittes 3 um die Raumachse 5 ragen die Enden 41, 42 des freien Endabschnittes 4 des ersten Substratabschnittes 3 jeweils seitlich frei zugänglich nach außen, siehe hierzu perspektivische Schrägansicht gemäß Figur 5b. Auf diese Weise lassen sich die faden- oder bandförmigen Stranggüter 8 unkompliziert fest an den Enden 41, 42 fügen. Die gegenüberliegenden Enden der Stranggüter 8 werden gleichsam der vorstehenden Ausführungsbeispiele im Bereich des zweiten Substratabschnittes 6 lösbar fest gefügt. Hierzu dienen Öffnungen 13, durch die die Endabschnitte der Stranggüter 8 geführt und entsprechend verknotet werden können.

Selbstverständlich ist es möglich, die in den vorstehenden Ausführungsbeispielen erläuterten Maßnahmen miteinander zu kombinieren.

### Bezugszeichenliste

- 1: medizinisches Implantat
- 2: Flächensubstrat
- 3: erster Substratabschnitt
- 4: freies Abschnittsende
- 41,42: Enden des Abschnittsendes
- 5: Raumachse
- 6: zweiter Substratabschnitt
- 61: stegartig ausgebildeter Flächenabschnitt
- 7: Verbindungsstruktur
- 8: Stranggut
- 9: Fügeverbindung
- 10: Verbindung
- 11: Öffnung
- 12: Fügeverbindung
- 13: Öffnung
- 14: Lasche
- 15: schlitzförmige Ausnehmung
- 16: seitlicher Bereich
- 17: Länge der schlitzförmigen Ausnehmung
- S: Schnittstelle
- H: zylinderförmiger Hohlraum
- E: Einschnürung
- A: Aufweitung
- W: Wickelerstreckung
- L: Manschettenlängserstreckung
- N: Nervenfaserbündel
- F: externe Kraft

## Patentansprüche

1. Vorrichtung zur extravasalen oder extraneuronalen Befestigung eines medizinischen Implantats (1) mit einem biokompatiblen Flächensubstrat (2), das einen in Art einer Wickelmanschette ausgebildeten ersten Substratabschnitt (3), der über ein freies Abschnittsende (4) verfügt, das durch Wickeln des ersten Substratabschnittes (3) um eine Raumachse (5) wenigstens einlagig lose von dem gewickelten ersten Substratabschnitt (3) radial überdeckt ist, sowie einen sich einstückig an den ersten Substratabschnitt (3) anschließenden zweiten, nicht um die Raumachse (5) gewickelten Substratabschnitt (6) aufweist, der mittel- oder unmittelbar mit einer von dem medizinischen Implantat (1) wegführenden Verbindungstruktur (7) verbindbar ist,
**dadurch gekennzeichnet, dass** im Bereich des Abschnittsendes (4) wenigstens ein Mittel (8) angebracht ist, das im gewickelten Zustand des ersten Substratabschnittes (3) um die Raumachse (5) mit dem ersten Substratabschnitt (3) und/oder dem zweiten Substratabschnitt (6) eine Fügeverbindung (9) eingeht.

2. Vorrichtung nach Anspruch 1,
**dadurch gekennzeichnet, dass** im gewickelten Zustand des ersten Substratabschnittes (3) zwischen dem Bereich des Abschnittsendes (4), an dem das Mittel (8) angebracht ist, und der Fügeverbindung (9) sich ein wenigstens einmal um die Raumachse (5) erstreckender Bereich des ersten Substratabschnittes (3) erstreckt.

3. Vorrichtung nach Anspruch 1 oder 2,
**dadurch gekennzeichnet, dass** die Fügeverbindung (9) lösbar fest ist.

4. Vorrichtung nach einem der Ansprüche 1 bis 3,
**dadurch gekennzeichnet, dass** die Fügeverbindung (9) eine Kraft- und/oder Formschlussverbindung ist.

5. Vorrichtung nach einem der Ansprüche 1 bis 4,
**dadurch gekennzeichnet, dass** das Mittel (8) ein Zugkräfte übertragendes Stranggut ist und einseitig fest im Bereich des Abschnittsendes (4) angebracht oder einstückig mit diesem verbunden ist.

6. Vorrichtung nach einem der Ansprüche 1 bis 5,
**dadurch gekennzeichnet, dass** das biokompatible Flächensubstrat (2) wenigstens eine das Flächensubstrat (2) vollständig durchsetzende Befestigungsöffnung (10) aufweist, durch die das Mittel (8) zum Zwecke der Fügeverbindung (9) geführt ist.

7. Vorrichtung nach einem der Ansprüche 1 bis 4,
**dadurch gekennzeichnet, dass** das Mittel (8) in Art einer Lasche ausgebildet ist, die den ersten Substratabschnitt (3) seitlich überragt, und
dass am ersten und/oder zweiten Substratabschnitt eine schlitzförmige Ausnehmung derart angeordnet und ausgebildet ist, so dass die Lasche in die schlitzförmige Ausnehmung unter Ausbildung der Fügeverbindung fügbar ist.

8. Vorrichtung nach einem der Ansprüche 1 bis 7,
**dadurch gekennzeichnet, dass** der erste Substratabschnitt (3) eine sich um die Raumachse (5) orientierte Wickelerstreckung (W) und eine axial zur Raumrichtung orientierte Manschettenlängserstreckung (L) aufweist, und
dass sich die Manschettenlängserstreckung (L) innerhalb des ersten Substratabschnittes (3) mit zunehmenden Abstand zum Bereich des Abschnittsendes (4) längs der Wickelerstreckung (W) stufenweise oder gleichmäßig verjüngt.

9. Vorrichtung nach einem der Ansprüche 1 bis 8,
**dadurch gekennzeichnet, dass** das freie Abschnittsende (4) des ersten Substratabschnittes (3) zwei sich längs des Abschnittsendes (4) gegenüberliegende Bereiche (41, 42) aufweist, an denen jeweils eines der Mittel (8) angebracht ist, und dass die Fügeverbindungen (9) beider Mittel (8) längs der Raumachse (5) im ersten Substratabschnitt (3) und/oder im zweiten Substratabschnitt (6) gegenüberliegend angeordnet sind.

## Claims

1. Device for extravasal or extraneuronal fastening of a medical implant (1) with a biocompatible surface substrate (2) biocompatible surface substrate having a first substrate portion (3) configured in the manner of a compression sleeve and has a free end portion (4) which by winding the first substrate portion (3) about a spatial axis (5) is loosely radially covered by at least one layer of the wound first substrate portion (3), and also a second substrate portion (6) which is attached integrally to the first substrate portion (3), is not wound about the spatial axis (5), and is connectable directly or indirectly to a connection structure (7) leading away from the medical implant (1),
**characterised in that** in the region of the end portion (4) at least one means (8) is applied, which, when first substrate portion (3) is in the wound state about the spatial axis (5) forms a joint connection (9) with the first substrate portion (3) and/or the second substrate portion (6).

2. Device according to claim 1,
**characterised in that** in the wound state of the first substrate portion (3), in the region of the portion end (4), on which the means (8) is applied, and the joint connection (9), an area of the first substrate portion (3) extends at least once about the spatial axis (5).

3. Device according to claim 1 or 2,
**characterised in that** the joint connection (9) is detachably fixed.

4. Device according to any one of claims 1 to 3, **characterised in that**in that the joint connection (9) is a frictional and/or interlocking connection.

5. Device according to any one of claims 1 to 4, **characterised in that** the means (8) is a strand material transmitting tensile forces and is unilaterally firmly attached in the region of the portion end (4) or integrally connected thereto.

6. Device according to any one of claims 1 to 5, **characterised in that** the biocompatible surface substrate (2) has at least one fastening opening (10) extending completely through the surface substrate (2), through which the means (8) is passed for the purpose of the joint connection (9).

7. Device according to any one of claims 1 to 4,
**characterised in that** the means (8) is designed in the manner of a tab which laterally projects beyond the first substrate portion (3) and
**in that** on the first and/or second substrate portion a slit-shaped recess is arranged and configured in such a way that the tab can be introduced into the slit-shaped recess to form the joint connection.

8. Device according to any one of claims 1 to 7,
**characterised in that** the first substrate portion (3) has a winding extension (W) orientated about the spatial axis (5) and a longitudinal cuff extension (L) orientated axially to the spatial direction
and **in that** the longitudinal cuff extension (L) within the first substrate portion (3) tapers in a step-like manner or evenly along the winding extension (W) with increasing distance to the region of the portion end (4) .

9. Device according to any one of claims 1 to 8, **characterised in that** the free portion end (4) of the first substrate portion (3) comprises two areas (41, 42) opposite each other along the portion end (4) on each of which one of the means (8) is applied, and
**in that** the joint connections (9) of both means (8) are arranged opposite each other along the spatial axis (5) in the first substrate portion (3) and/or in the second substrate portion (6).

## Revendications

1. Dispositif de fixation extravasal ou extraneuronal d'un implant médical (1) avec un substrat superficiel biocompatible (2), la première section de substrat (3) constituée à la manière d'un brassard, qui dispose d'une extrémité de section libre (4), qui est radialement recouverte au moins en une couche non serrée par la première section de substrat (3) enroulée par enroulement de la première section de substrat (3) autour d'un axe dans l'espace (5), comporte une deuxième section de substrat (6) non enroulée autour de l'axe dans l'espace (5) se raccordant en une pièce à la première section de substrat (3), qui peut être raccordée indirectement ou directement à une structure de liaison (7) s'écartant de l'implant médical (1),
**caractérisé en ce que** dans la zone de l'extrémité de section (4) est au moins disposé un moyen (8), qui à l'état enroulé de la première section de substrat (3) autour de l'axe dans l'espace (5) engendre une liaison de jonction (9) avec la première section de substrat (3) et/ou la deuxième section de substrat (6).

2. Dispositif selon la revendication 1,
**caractérisé en ce qu'**à l'état enroulé de la première section de substrat (3), entre la zone de l'extrémité de section (4), sur laquelle est disposé le moyen (8) et la liaison de jonction (9), s'étend une zone de la première section de substrat (3) s'étendant au moins une fois autour de l'axe dans l'espace (5).

3. Dispositif selon la revendication 1 ou 2, **caractérisé en ce que** la liaison de jonction (9) est fixe pouvant être amovible.

4. Dispositif selon l'une quelconque des revendications 1 à 3,
**caractérisé en ce que** la liaison de jonction (9) est une liaison par conformité de force et/ou de forme.

5. Dispositif selon l'une quelconque des revendications 1 à 4,
**caractérisé en ce que** le moyen (8) est un matériau allongé transmettant une force de traction et est disposé d'un côté fixe dans la zone de l'extrémité de section (4) ou relié en une seule pièce à celle-ci.

6. Dispositif selon l'une quelconque des revendications 1 à 5,
**caractérisé en ce que** le substrat superficiel biocompatible (2) comporte au moins une ouverture de fixation (10) traversant complètement le substrat superficiel (2) à travers laquelle est passé le moyen (8) en vue de la liaison de jonction (9).

7. Dispositif selon l'une quelconque des revendications 1 à 4,
**caractérisé en ce que** le moyen (8) est constitué sous la forme d'une languette, qui dépasse latéralement la première section de substrat (3), et
**en ce que** sur la première et/ou la deuxième section de substrat est disposé et constitué un évidement en forme de fente de telle manière que la languette peut être assemblée dans l'évidement en forme de fente en formant la liaison d'assemblage.

8. Dispositif selon l'une quelconque des revendications 1 à 7,
**caractérisé en ce que** la première section de substrat (3) comporte une extension d'enroulement (W) orientée autour de l'axe dans l'espace (5) et une extension longitudinale de brassard (L) orientée axialement à la direction spatiale, et
**en ce que** l'extension longitudinale de brassard (L) se réduit progressivement ou de façon uniforme à l'intérieur de la première section de substrat (3) le long de l'extension d'enroulement (W) avec une distance croissante par rapport à la zone de l'extrémité de section (4).

9. Dispositif selon l'une quelconque des revendications 1 à 8,
**caractérisé en ce que** l'extrémité de section libre (4) de la première section de substrat (3) comporte deux zones (41, 42) opposées le long de l'extrémité de section (4) sur lesquelles est respectivement disposé un des moyens (8) et **en ce que** les liaisons de jonction (9) des deux moyens (8) sont disposés opposés le long de l'axe dans l'espace (5) dans la première section de substrat (3) et/ou dans la deuxième section de substrat (6) .
